# EUROPÄISCHE PATENTSCHRIFT

(11) **EP 2 892 584 B1**
(45) Veröffentlichungstag und Bekanntmachung des Hinweises auf die Patenterteilung: **14.06.2017**
(21) Anmeldenummer: 13765287.1
(22) Anmeldetag: 04.09.2013
(51) Int. Cl.: A61M 1/14, A61M 1/34

(54) **BLUTBEHANDLUNGSVORRICHTUNG MIT VORRICHTUNG ZUM QUANTIFIZIEREN UND DARSTELLEN EINES ZEITPUFFERS EINES PATIENTEN**
BLOOD TREATMENT APPARATUS WITH A DEVICE FOR QUANTIFYING AND DISPLAYING A TIME BUFFER OF A PATIENT
APPAREIL DE TRAITEMENT DU SANG AVEC DISPOSITIF DE QUANTIFICATION ET DE REPRESENTATION D'UN DELAI-TAMPON D'UN PATIENT

(30) Priorität: 04.09.2012 DE 102012017432; 04.09.2012 US 201261696333 P
(43) Veröffentlichungstag der Anmeldung: 15.07.2015
(73) Patentinhaber: Fresenius Medical Care Deutschland GmbH, 61352 Bad Homburg (DE); Fresenius Medical Care Italia S.p.A., 26020 Palazzo Pignano (Cremona) (DE)
(72) Erfinder: DUELSNER, Erik, 61197 Florstadt (DE); MARAZZI, Marcello, 26025 Pandino (CR) (IT); WEHMEYER, Wolfgang, 72076 Tuebingen (DE)
(74) Vertreter: Bobbert & Partner Patentanwälte PartmbB
(86) Internationale Anmeldenummer: PCT/EP2013/002661
(87) Internationale Veröffentlichungsnummer: WO 2014/037106

(56) Entgegenhaltungen:
- US-A- 5 656 153
- US-A1- 2004 267 575
- US-A1- 2006 004 606
- US-A1- 2009 281 825
- US-A1- 2012 029 937

## Beschreibung

Die vorliegende Erfindung betrifft eine Blutbehandlungsvorrichtung mit einer Vorrichtung zum Quantifizieren und zum Darstellen eines Zeitpuffers eines Patienten gemäß Anspruch 1.

Aus der Praxis sind Blutbehandlungszentren wie Dialysezentren bekannt, in welchen täglich mit einer begrenzten Anzahl von Behandlungsvorrichtungen, wie etwa Dialysemaschinen, eine Vielzahl von Patienten behandelt werden. In vielen dieser Zentren ist es dabei erforderlich, die Patienten im Mehrschichtbetrieb zur Behandlung einzubestellen. Zum Erzielen eines reibungslosen Betriebs eines solchen Zentrums bei gleichzeitig bestmöglicher Behandlung jedes Patienten ist es wichtig, bestimmte Zeiten wie den geplanten Schichtwechsel oder das geplante Ende der anstehenden Behandlungssitzung möglichst einzuhalten. Das Einhalten dieser Zeiten kann ferner zum Einsparen von Kosten etwa für wartende (Sammel-) Taxis beitragen. Zum Einhalten dieser Zeiten ist es erforderlich, die individuelle Eile (welche auch über einen "Zeitpuffer" ausgedrückt werden kann), mit der ein konkreter, eben im Behandlungszentrum eingetroffener Patient mit der anstehenden Behandlung beginnen muss, zu kennen. Es kann auch erforderlich sein, zu wissen, wie eilig die Behandlungssitzung dieses Patienten zu beginnen ist angesichts der Eile weiterer Patienten, die ebenfalls auf ihren Behandlungsbeginn warten und ebenfalls vom medizinischen Personal zur Behandlung vorbereitet werden müssen (etwa durch das Legen eines Zugangs, durch Messung des Körpergewichts, usw.). Nicht alle Informationen, die zum Erkennen der individuellen Eile erforderlich sind, sind dem medizinischen Personal bekannt.

Aus der US 2006/0004606 A1 ist ein Zeitmanagement für medizinische Anwendung, insbesondere im Krankenhausumfeld, bekannt.

Aus der US 2004/0267575 A1 sind Verfahren und System zum Überwachen medizinischer Untersuchungen und/oder Behandlungen bekannt.

Aus der US 201270029937 A1 ist eine Dialysevorrichtung bekannt.

Eine Aufgabe der vorliegenden Erfindung ist es, eine Blutbehandlungsvorrichtung, die eine Vorrichtung zum Quantifizieren und Darstellen eines Zeitpuffers aufweist, vorzuschlagen.

Die erfindungsgemäße Aufgabe wird durch eine Blutbehandlungsvorrichtung mit den Merkmalen des Anspruchs 1 gelöst.

Erfindungsgemäß wird somit eine Blutbehandlungsvorrichtung mit einer Vorrichtung vorgeschlagen, welche konfiguriert ist zum Quantifizieren oder Ermitteln und zum Darstellen eines Zeitpuffers oder einer Eilbedürftigkeit wenigstens eines Patienten vor und/oder während einer Blutbehandlungssitzung.

Die Vorrichtung weist wenigstens eine Einrichtung zum Einlesen (das Einlesen kann hierin in einigen erfindungsgemäßen Ausführungsformen auch als ein Auslesen verstanden werden) einer - in aller Regel vom Arzt verschriebenen und/oder einer angestrebten - Behandlungsdauer in die Vorrichtung auf. Die Behandlungsdauer gibt an, über welche Zeit der Patient oder sein Blut während der betrachteten Blutbehandlungssitzung behandelt werden soll.

Das Einlesen der Behandlungsdauer kann beispielsweise aus einer Patientenkarte oder einer Datenbank mit entsprechenden Speicherinhalten eingelesen werden. Unter einem Einlesen kann erfindungsgemäß beispielsweise auch ein Übertragen von einer manuell eingegebenen Behandlungsdauer zwischen einem ersten Unter einer Behandlungsdauer kann erfindungsgemäß eine Netto-Behandlungsdauer oder eine reine Behandlungsdauer des Patienten verstanden werden, zu welcher die Dauer von Aufrüst- und Abrüsttätigkeiten, sowie Ruhe- oder Pausenzeiten der Behandlungsvorrichtung, während welcher eine Blutreinigung nicht erfolgt oder nicht voranschreitet, nicht zählen. Die Behandlungsdauer kann beispielsweise der vom Arzt vorgegebenen oder verschriebenen Behandlungsdauer entsprechen. Die Behandlungsdauer kann beispielsweise jene Dauer (beispielsweise in Minuten gerechnet) sein, während welcher die Behandlungsvorrichtung effektiv oder medizinisch wirksam in der von zur Blutreinigung vorgesehenen Weise auf das Blut einwirkt.

Die Vorrichtung weist ferner wenigstens eine Einrichtung auf zum wenigstens einmaligen Einlesen eines - beispielsweise vom medizinische Personal vorgesehenen oder angestrebten - Endzeitpunkts der Blutbehandlungssitzung in die Vorrichtung.

Unter einem Endzeitpunkt der Blutbehandlungssitzung kann erfindungsgemäß ein Wunsch-Endzeitpunkt für die Blutbehandlungssitzung zu verstehen sein. Der Wunsch-Endzeitpunkt kann vom medizinischen Personal oder vom Patienten oder durch äußere Umstände vorgegeben sein. Der Endzeitpunkt kann beispielsweise durch ein Schichtende bei der Betreuung von Patienten in Schichten, durch einen sich an die Blutbehandlungssitzung anschließenden Termin des Patienten, durch ein für einen bestimmten Zeitpunkt bestelltes (Sammel-)Taxi oder andere Fristen oder Termine bestimmt sein.

Die Vorrichtung weist des Weiteren wenigstens eine Einrichtung zum wenigstens einmaligen Berechnen oder Ermitteln oder Klassifizieren eines patientenindividuellen Zeitpuffers oder einer patientenindividuellen Eilbedürftigkeit auf. Der Zeitpuffer oder die Eilbedürftigkeit wird basierend wenigstens oder ausschließlich auf Basis der aktuellen Uhrzeit, welche dem frühest möglichen Startzeitpunkt der Behandlungssitzung entspricht, der Behandlungsdauer und dem Endzeitpunkt ermittelt oder berechnet.

Die Vorrichtung weist schließlich wenigstens eine Einrichtung auf zum wenigstens einmaligen Ausgeben oder Anzeigen eines Signals, welches den Zeitpuffer angibt oder die Eilbedürftigkeit oder hierfür codiert.

Unter einem Zeitpuffer ist erfindungsgemäß eine Angabe darüber zu verstehen, wie viel Zeit dem Patienten oder dem betreuenden medizinischen Personal verbleibt, um mit der Behandlung (im Sinne der Behandlungsdauer) des Patienten zu beginnen, derart, dass der Patient bis zum (angestrebten und eingelesenen) Endzeitpunkt über eine Zeitspanne behandelt wurde, welche der (angestrebten und eingelesenen) Behandlungsdauer entspricht. Ergibt die Berechnung des Zeitpuffers, dass noch eine gewisse Zeit verstreichen darf, bis mit der Behandlung angefangen werden muss und die Behandlung mit der verschriebenen Behandlungsdauer des Patienten trotzdem noch immer bis zum Endzeitpunkt erfolgen kann, so kann man von einem positiven Zeitpuffer sprechen. In diesem Fall besteht keine (besondere) Eile, mit der Behandlung zu beginnen.

Ergibt die Berechnung hingegen, dass es angesichts der aktuellen (Uhr-)Zeit nicht mehr möglich ist, dem Patienten bis zum Endzeitpunkt eine Behandlung mit der gewünschten Behandlungsdauer angedeihen zu lassen, so kann man von einem negativen Zeitpuffer sprechen. Der Begriff Zeitpuffer, wie er hierin verwendet wird, umfasst somit sowohl einen Zeitüberschuss (positiver Zeitpuffer) als auch einen Zeitmangel (negativer Zeitpuffer). Im letzteren Fall besteht für den Fall, dass der Patient am Behandlungstag eine Behandlung im Umfang der gesamte Behandlungsdauer (wie vorstehend definiert) erhalten muss, große Eile mit dem Beginn der Behandlung zu beginnen. Andernfalls verzögert sich die Behandlung noch weiter über den Endzeitpunkt hinaus, das Taxi muss noch länger warten, der sich anschließende Termin oder der Beginn der an derselben Behandlungsvorrichtung geplanten Behandlung des nächsten Patienten verschiebt sich, usw. Der Patient liegt bei einem negativen Zeitpuffer, kurz gesprochen, hinter dem Zeitplan.

Dabei ist die Einschätzung, ob ein Zeitpuffer positiv oder negativ ist, und/oder ob eine Eilbedürftigkeit hoch oder niedrig ist, und/oder wie eine eventuell vorgenommene Klassifizierung vorgenommen wird, ebenso festlegbar, wie das Signal, mit welchem der Zeitpuffer, die Eilbedürftigkeit oder die jeweilige Klasse hiervon angegeben wird. So kann eine hohe Eilbedürftigkeit beispielsweise mit einem roten Warnsignal auf einem Monitor angegeben werden, eine niedrige hingegen beispielsweise mit einem grünen Signal. Dabei bleibt es aber dem Anwender oder Hersteller überlassen festzulegen, welcher Zeitpuffer noch als grün angezeigt wird, und wann ein Zeitpuffer bereits mit einer roten Markierung einher gehen muss.

Die Begriffe Zeitpuffer und Eilbedürftigkeit sind hierin als Synonyme zu verstehen, welche aus Gründen des besseren Verständnis nebeneinander verwendet werden. Sie sind hierin gegeneinander austauschbar.

Wenn hierin von einer hohen oder geringen Eilbedürftigkeit, einem positiven oder negativen Zeitpuffer die Rede ist, so gilt in bestimmten erfindungsgemäßen Ausführungsformen der vorliegenden Erfindung, dass die Adjektive wie beispielsweise "hohe", "geringe", "positiv", "negativ" - aber auch alle übrigen wie "groß", "klein" oder dergleichen - aufgrund einer zuvor anhand vorbestimmter Kriterien festgelegten, entsprechenden Klassifizierung als "hohe", "geringe" usw. einzustufen sind. Diese Begriffe sind somit bestimmt, da sie durch vorbestimmte Kriterien messbar sind und diese wiedergeben.

Unter einem Zeitpuffer kann erfindungsgemäß auch ein Behandlungsstatus, also eine Ausgabe darüber, welche Schritte der Behandlungssitzung am Patienten bereits durchgeführt oder erfolgt sind, (selbst wenn die Behandlung noch nicht begonnen hat oder der Patient beispielsweise noch nicht mit der Behandlungsvorrichtung verbunden wurde) zu verstehen sein. Diese Begriffe können, wie hierin verwendet, gegeneinander ausgetauscht werden.

Unter einem Klassifizieren kann ein vorab festgelegtes Vorsehen von Klassen (beispielsweise hoch, mittel, niedrig; oder eilt sehr, eilt wenig; usw.) zu verstehen sein, zu welchen ein Zeitpuffer zugeordnet werden kann. Beim Klassifizieren wird der Zeitpuffer nicht bis ins Letzte quantifizierbar angegeben, sondern einer Klasse aus einer Vielzahl von möglichen Klassen an Berechnungs- oder Ermittelungsergebnissen zugeordnet.

Unter einem Codieren des Zeitpuffers kann ein Klassifizieren zu verstehen sein. Beispielsweise kann ein positiver Zeitpuffer mittels eines grünen Farbsignals auf einer Anzeige wie einem Display angezeigt werden. Ein negativer Zeitpuffer oder eine hohe Eilbedürftigkeit kann mittels eines roten Farbsignals angezeigt werden. Abstufungen in Farbe, Form und/oder Erscheinung sind erfindungsgemäß beliebig möglich. Andererseits kann das Signals auch nicht-codiert angegeben werden. Hierbei kann das Berechnungs- oder Ermittelungsergebnis beispielsweise als konkreter Zahlenwert (beispielsweise in Minuten, beispielsweise mit negativem Vorzeichen für einen negativen Zeitpuffer und mit positivem Vorzeichen für einen positiven Zeitpuffer) angeben werden.

Die erfindungsgemäße Blutbehandlungsvorrichtung weist wenigstens eine hierin offenbarte Vorrichtung auf.

Das offenbarte Verfahren dient dem Quantifizieren und Darstellen des Zeitpuffers oder dem Ermitteln und Darstellen des Zeitpuffers wenigstens eines Patienten bei, vor und/oder während einer Blutbehandlungssitzung. Das Verfahren wird mittels einer entsprechend konfigurierten Vorrichtung ausgeführt, insbesondere mittels einer Vorrichtung.

Das offenbarte Verfahren umfasst ein mittels der Vorrichtung erfolgendes Einlesen einer Behandlungsdauer.

Das offenbarte Verfahren umfasst ferner ein wenigstens einmaliges Einlesen eines Endzeitpunkts der Blutbehandlungssitzung mittels der Vorrichtung.

Zudem umfasst das offenbarte Verfahren ein wenigstens einmaliges Berechnen, Ermitteln oder Klassifizieren eines Zeitpuffers mittels der Vorrichtung. Das Berechnen oder Ermitteln basiert wenigstens oder ausschließlich auf der aktuellen (Uhr-)Zeit, der Behandlungsdauer und des Endzeitpunkts oder bezieht diese Werte beim Berechnen, Ermitteln oder Klassifizieren ein.

Das offenbarte Verfahren umfasst schließlich ein wenigstens einmaliges Ausgeben eines Signals, welches den Zeitpuffer angibt oder hierfür codiert, durch die Vorrichtung.

Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In bestimmten erfindungsgemäßen Ausführungsformen wird unter dem Zeitpuffer die Differenz "Endzeitpunkt - Behandlungsdauer - (Uhr-)Zeit" oder jede andere Rechenvorschrift verstanden, welche die vorstehende Beziehung aufweist. Soll die Behandlungssitzung beispielsweise um 12:00 beendet sein (Endzeitpunkt) und 3:00 Stunden dauern (Behandlungsdauer), so ist der Zeitpuffer um 9:00 (Zeit) genau 0, um 8:00 Uhr würde der Zeitpuffer noch eine Stunde betragen. Je nach Klassifizierung (und Kodierung) könnte der Zeitpuffer um 8:00 mit "60 min" oder mit einem grünen Signal dem Personal darstellt werden. Es bedarf keiner Erläuterung dazu, dass sich der Zeitpuffer stets verändert. So könnte der Zeitpuffer im vorstehenden Beispiel um 8:30 bereits mit einem gelben Signal, und um 8:50 gar mit einem roten Signal dargestellt werden.

In bestimmten erfindungsgemäßen Ausführungsformen weist die Vorrichtung eine Einrichtung zum Übermitteln der Behandlungsdauer und/oder des eingelesenen Endzeitpunkts der Blutbehandlungssitzung an eine Blutbehandlungsvorrichtung auf.

In manchen erfindungsgemäßen Ausführungsformen erfolgt die Übermittlung der Behandlungsdauer und/oder des eingelesenen Endzeitpunkts der Blutbehandlungssitzung an die Blutbehandlungsvorrichtung mittels eines Netzwerkes. Ein Netzwerk kann ein Speichermedium oder einen Computer mit einem anderen Computer verbinden. Die Bluthandlungsvorrichtung kann einen Computer, eine Speichereinrichtung oder einen Rechner aufweisen, welche jeweils konfiguriert ist, um Daten von einem Speicher mittels des Netzwerkes einzulesen.

In einigen erfindungsgemäßen Ausführungsformen erfolgt die Übermittlung der Behandlungsdauer und/oder des eingelesenen Endzeitpunkts der Blutbehandlungssitzung an die Blutbehandlungsvorrichtung durch direktes Einlesen mittels eines Computers in der Blutbehandlungsvorrichtung aus einem Speicher der mobil mitgeführten, elektronischen Patientenkarte.

In manchen erfindungsgemäßen Ausführungsformen werden Schritte der Blutbehandlungssitzung unmittelbar durch das Einlesen der Behandlungsdauer und/oder dem Übermitteln des eingelesenen Endzeitpunkts der Blutbehandlungssitzung initiiert oder aktiviert.

In bestimmten erfindungsgemäßen Ausführungsformen weist die Vorrichtung eine Einrichtung auf, welche konfiguriert ist zum Bestimmen oder Verschieben des Zeitpunkts des erforderlichen Beginns der Blutbehandlungssitzung (der Beginn der Blutbehandlungssitzung kann auch als Startzeitpunkt bezeichnet werden) oder zum Bestimmen oder Errechnen des Zeitpuffers. Dabei werden die zur Vorbereitung der Blutbehandlung oder während der Blutbehandlungssitzung erforderlichen Schritte, während welcher keine Behandlung des Bluts erfolgt, und/oder die erforderlichen Zeiten mit berücksichtigt.

Erforderliche Schritte für die weitere Durchführung der Blutbehandlung können beispielsweise sein: Konnektieren des Patienten an die Blutbehandlungsvorrichtung, Messen des Blutdrucks des Patienten vor Beginn der Behandlung, automatisch oder nicht automatisch durchgeführte Funktionstests der Blutbehandlungsvorrichtung, welche vor oder während der Blutbehandlungssitzung durchgeführt werden, sowie weitere notwendige Vorbereitungsschritte zum Starten oder Beenden der Blutbehandlungssitzung wie Primen, Spülen Reinigen von Disposables. Mittels dieser Einrichtung wird der Startzeitpunkt unter Berücksichtung der Zeiten zur Durchführung der weiteren Schritte angepasst. Dies kann durch Addieren eines Default-Wertes oder durch Addieren eines flexiblen Zeitwerts zur Behandlungsdauer geschehen, welche diese Zeiten jeweils als Summe beinhalten. Der Beginn oder Startzeitpunkt der Blutbehandlungssitzung erfolgt somit früher und die Dauer der Behandlungssitzung ist um den Default-Wert oder um den flexiblen Wert länger als die Behandlungsdauer. Anders ausgedrückt wird der Zeitpuffer bei Berücksichtung der weiteren erforderlichen Schritte de facto verringert (oder als verringert ermittelt) und entsprechend dargestellt oder angezeigt. Die vorstehend genannten Verfahren, mittels welcher die für die weiteren Schritte einzuplanenden Zeitdauern in Anzeigen oder Ausgaben einfließen, sind Beispiele für eine Berücksichtung der weiteren Schritte und/oder ihrer Dauern.

In manchen erfindungsgemäßen Ausführungsformen weist die Vorrichtung eine Einrichtung auf, welche zum Korrigieren des Zeitpuffers und/oder seiner Darstellung um die zur Durchführung der weiteren Schritte zusätzlich erforderliche Zeit konfiguriert ist. Eine solche Korrektur kann in einigen erfindungsgemäßen Ausführungsformen regelmäßig oder auf Hinweis oder Abfrage durch die Behandlungsvorrichtung oder das Personal erfolgen.

In bestimmten erfindungsgemäßen Ausführungsformen weist die Vorrichtung eine Einrichtung auf, welche zum Einlesen einer vorgesehenen Gesamtbehandlungsdauer des Patienten konfiguriert ist. Unter der Gesamtbehandlungsdauer kann erfindungsgemäß eine über eine vorbestimmte Anzahl von Behandlungssitzungen aufsummierte Behandlungsdauer zu verstehen sein. Die Gesamtbehandlungsdauer unterscheidet sich in diesen erfindungsgemäßen Ausführungsformen von der wie oben definierten Behandlungsdauer allein dadurch, dass sie sich auf eine Vielzahl x von Behandlungssitzungen bezieht, während sich die Behandlungsdauer auf die jeweils anstehende oder gerade durchgeführte Behandlungssitzung bezieht. Die Gesamtbehandlungsdauer ist somit, vereinfacht gesprochen, ebenfalls eine vom Arzt verschriebene Zeitdauer, während welcher das Blut des Patienten verteilt auf mehrere Behandlungssitzungen wirksam behandelt werden soll. Eine Gesamtbehandlungsdauer kann sich beispielsweise auf einen Zeitraum von einer Woche beziehen. Eine Gesamtbehandlungsdauer kann beispielsweise die aufsummierten Behandlungsdauern von drei bis fünf oder mehr Behandlungssitzungen umfassen.

Die Vorrichtung weist weiterhin eine Einrichtung zum Aufsummieren der tatsächlich bislang erfolgten Dauern auf, während welcher der Patient im Rahmen der vorbestimmten Anzahl - beispielsweise mit einer Anzahl von x-1 - von aufeinander folgend erfolgter, abgeschlossener Behandlungssitzungen medizinisch wirksam behandelt wurde.

Die Vorrichtung weist ferner eine Einrichtung zum Festlegen oder Abändern der eingelesenen Behandlungsdauer der anstehenden Blutbehandlungssitzung unter Berücksichtigung der eingelesenen Gesamtbehandlungsdauer und der aufsummierten Dauern der erfolgten Behandlungen auf. Die Vorrichtung kann die Behandlungsdauer der Blutbehandlungssitzung aufgrund von bereits durchgeführten Behandlungssitzungen festlegen. Wurde der Patient beispielsweise in wenigstens einer der vorangegangenen Behandlungssitzungen länger behandelt, als es der Gesamtbehandlungsdauer geteilt durch die vorbestimmte Anzahl der Behandlungssitzungen entspricht (gleiche Behandlungsdauern pro Sitzung hier einmal unterstellt), so kann die Dauer der aktuellen Behandlungssitzung ggf. gegenüber der für diese Behandlungssitzung verschriebenen Behandlungsdauer verkürzt werden. Dies mag der Zeitplanung des Patienten und/oder der Organisationsablauf des Behandlungszentrum im Einzelfall entgegen kommen. Ebenso kann die Dauer der tatsächlich durchgeführten Behandlung der aktuellen Behandlungssitzung auch einmal verlängert werden. Dies kann dem Patienten einen Behandlungs"puffer" gewähren, um welchen er bei einer der folgenden Behandlungssitzungen kürzer als die verschriebene Behandlungsdauer an der Behandlungsmaschine verbleiben muss.

Unter einer "Berücksichtigung der eingelesenen Gesamtbehandlungsdauer" kann erfindungsgemäß das Bilden einer Differenz, oder eine Rechenvorschrift verstanden werden, in welcher diese Differenz mathematisch aufgeht oder deren Teil sie ist. So kann als Gesamtdauer festgelegt sein, dass der Patient während drei aufeinander folgender Behandlungssitzungen insgesamt 12 Stunden (Gesamtbehandlungsdauer) behandelt werden soll. Er wurde in den vergangenen beiden Behandlungssitzung hiervon abweichend jedoch bereits 8,5 Stunden (aufsummierte Dauern) behandelt. Daher kann die eingelesene Behandlungsdauer von 4 Stunden für die nun anstehende Behandlungssitzung als 12 h - 8,5 h = 3,5 h festgelegt bzw. abgeändert werden.

In manchen erfindungsgemäßen Ausführungsformen kann eine derart abgeänderte Behandlungsdauer anstelle der eingelesenen Behandlungsdauer zur Ermittelung des Zeitpuffers herangezogen werden. Die erfindungsgemäße Vorrichtung ist in diesen Ausführungsformen entsprechend konfiguriert oder weist eine solche Einrichtung auf. Aus einem negativen Zeitpuffer kann in erfindungsgemäßen Ausführungsformen, in denen eine derartige Abänderung erfolgt, deren Umfang aufgrund von Vorgaben beschränkt ist, unter Umständen durchaus ein positiver Zeitpuffer werden.

In bestimmten erfindungsgemäßen Ausführungsformen weist die Vorrichtung eine Einrichtung auf, welche zum Eingeben einer gegenüber der eingelesenen Behandlungsdauer verkürzten Behandlungsdauer - aufgrund oder bei Ermitteln eines beispielsweise negativen Zeitpuffers oder einer hohen Eilbedürftigkeit - aber auch einer verlängerten Behandlungsdauer mittels der Vorrichtung konfiguriert ist. Die eingelesene Behandlungsdauer kann im Einzelfall zu lang sein, um aufgrund der fortgeschrittenen aktuellen (Uhr-)Zeit der Blutbehandlungssitzung und des festgelegten Endzeitpunkts eingehalten werden zu können, wobei oder weshalb dem Patient ein negativer Zeitpuffer zugeschrieben wird. Zum Einhalten des Endzeitpunkts, was in bestimmten Fällen erforderlich sein kann, ist die Einrichtung derart konfiguriert, dass die Behandlungsdauer (gegenüber der eingelesenen Behandlungsdauer) erneut eingegeben oder eingelesen und dabei verkürzt wird oder wirken kann oder als verkürzt übernommen wird, um den festgelegten Endzeitpunkt einzuhalten. Ebenso kann vorgesehen sein, die Behandlungsdauer zu verlängern, um die Zeit bis zum Endzeitpunkt möglichst für die Behandlung über die eingelesene Behandlungsdauer hinaus zu nutzen.

Die Vorrichtung kann eine Einrichtung oder Instanz aufweisen, welche sicherstellt, dass die neue, verkürzte Behandlungsdauer eine bestimmte Mindest-Behandlungsdauer nicht unterschreitet, selbst wenn die Blutbehandlungssitzung dann - bereits frühzeitig erkennbar - nicht bis zum Endzeitpunkt beendet werden kann.

In bestimmten erfindungsgemäßen Ausführungsformen weist die Vorrichtung eine Einrichtung zum mehrfachen Abfragen oder Identifizieren eines Fortgangs der Behandlung des Patienten, kurz: Abfrageeinrichtung, auf. Die Abfrage mittels der entsprechend konfigurierten Abfrageeinrichtung erfolgt während der Behandlungssitzung durch Auslesen oder Identifizieren von durch die Behandlungsvorrichtung vorgenommenen oder initiierten Unterbrechungen der Behandlung des Patienten. Derartige Unterbrechungen sind als ein Aussetzen der Behandlung zu verstehen. Während einer solchen Unterbrechung wird der Patient von der Behandlungsvorrichtung nicht medizinisch behandelt. Während derartiger Unterbrechungen wird die (Rest-) Behandlungsdauer nicht geringer.

Die Vorrichtung weist in diesen erfindungsgemäßen Ausführungsformen weiterhin eine Einrichtung zum Ermitteln oder Abschätzen der Gesamtzeitdauer der identifizierten Unterbrechungen (oder von einigen der identifzierten Unterbrechungen, ggf. alle, die eines bestimmten Typs sind, einer bestimmten Klasse zugehören, eine Mindestdauer aufweisen, oder dergleichen) der Behandlung mittels der Abfrageeinrichtung auf.

Ferner weist die Vorrichtung in diesen Ausführungsformen eine Einrichtung auf, welche konfiguriert ist zum Berechnen, Ermitteln oder Klassifizieren des Zeitpuffers oder der Eilbedürftigkeit. Dabei werden wenigstens die aktuelle Zeit, die Behandlungsdauer, die Gesamtzeitdauer der identifizierten oder akkumulierten Unterbrechungen und der Endzeitpunkt berücksichtigt oder in die Berechnung, Ermittlung oder Klassifizierung mit einbezogen. Bei der Einrichtung kann es um eine der bereits oben genannten Einrichtungen handeln.

In manchen erfindungsgemäßen Ausführungsformen weist die Vorrichtung eine Einrichtung auf, welche konfiguriert ist zum Vergleichen von Zeitpuffern oder Eilbedürftigkeiten von mehreren Patienten miteinander und zum Festlegen einer Behandlungsreihenfolge dieser Patienten aufgrund des Ergebnisses des Vergleichs.

Das Ergebnis des Vergleichs kann wiederum mittels geeigneter Signale angezeigt oder ausgegeben werden. Dies kann beispielsweise in Form einer Reihenfolge geschehen, in welcher sich das Personal den einzelnen Patienten zuwenden sollte. Das Ergebnis des Vergleichs kann alternativ unter einem Zusammenfassen von Patienten in Dringlichkeitsklassen geschehen, oder durch andere Möglichkeiten.

In manchen erfindungsgemäßen Ausführungsformen weist die Vorrichtung eine Einrichtung auf, welche konfiguriert ist zum erneuten Festlegen des Endzeitpunkts und/oder der Behandlungsdauer aufgrund oder bei Erkennen eines Zeitpuffers, welcher unter einen vorgegebenen Schwellenwert fällt.

Jede der hierin genannten Einrichtungen der Vorrichtung kann eigens eingerichtet, konfiguriert oder auf andere Weise vorbereitet sein, um den jeweiligen Verfahrensschritt auszuführen, auch wenn dies nicht eigens hierin ausgeführt ist.

Jede der hierin genannten Einrichtungen der Vorrichtung kann eigens eingerichtet, konfiguriert oder auf andere Weise vorbereitet sein, um mehr als nur einen der hierin genannten Verfahrensschritte auszuführen.

Merkmalskombinationen von offenbarten Verfahren ergeben sich auch aus den hier angehängten Ansprüchen, auf welche hier vollumfänglich verwiesen wird, und welche allein zur Vermeidung von Wiederholungen hier nicht aufgeführt werden.

Die zu der Vorrichtung getroffenen Feststellungen und Definitionen treffen immer dort, wo dies für den Fachmann zu keinem Widerspruch führt, auch auf die offenbarten Verfahren zu.

Manche oder alle erfindungsgemäßen Ausführungsformen können einen, mehrere oder alle der oben und/oder im Folgenden genannten Vorteile aufweisen.

Mit dem erfindungsgemäßen Quantifizieren und Darstellen von Zeitpuffern können folgende Vorteile hinsichtlich des Patientenmanagements erzielt werden: verringerte Wartezeiten für Patienten bei dem An- und Abtransport zur Blutbehandlung (sogenannte Taxi-Wartezeiten), verbesserter und vorhersagbarer Workflow des anwesenden medizinischen Betreuungspersonals, geringerer Stresslevel beim Personal und den Patienten und, daher, geringere hygienische Risiken bei der Blutbehandlung, und Verlassen der Patienten aus dem Blutbehandlungsraum innerhalb eines vorgegebenen Zeitfensters, so dass der Blutbehandlungsraum nach hygienischen und effizienten Regeln gereinigt werden kann.

Mit der Vorrichtung und dem offenbarten Verfahren kann die Lebensqualität der Patienten vorteilhaft dahingehend verbessert werden, dass sie nur die notwendigen Zeiten im Blutbehandlungsraum und im Blutbehandlungszentrum verweilen. Dies führt überdies zu geringeren Kosten für das Behandlungszentrum.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- **Fig. 1**: zeigt schematisch vereinfacht eine Vorrichtung zum Quantifizieren und Darstellen eines Zeitpuffers, eingebettet in eine erfindungsgemäße Blutbehandlungsvorrichtung; und
- **Fig. 2**: zeigt schematisch in Flussdiagramm die Schritte des offenbarten Verfahrens zum Quantifizieren und Darstellen eines Zeitpuffers in einer exemplarischen Ausführungsform.

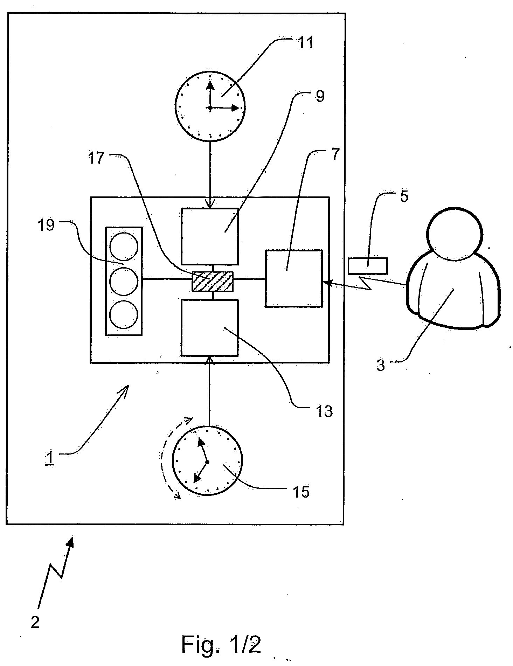

**Fig. 1** zeigt schematisch vereinfacht eine Vorrichtung 1, welche Teil einer erfindungsgemäßen Blutbehandlungsvorrichtung 2 ist, zum Quantifizieren und Darstellen eines Zeitpuffers eines Patienten 3 bei einer Blutbehandlungssitzung.

Eine Behandlungsdauer 15 für die Blutbehandlungssitzung des Patienten 3 ist in diesem Ausführungsbeispiel auf einer Patientenkarte 5 gespeichert, die der Patient 3 beispielsweise zur Blutbehandlungssitzung mitbringt. Die Behandlungsdauer 15 wurde zuvor auf der Patientenkarte 5 gespeichert, beispielsweise in der Arztpraxis des behandelnden Nephrologen. Die Behandlungsdauer 15 wird mittels einer Einrichtung 7, einem Lesegerät für die Patientenkarte 5, aus der Patientenkarte 5 eingelesen. Die Einrichtung 7 ist Teil der Vorrichtung 1.

Weiterhin weist die Vorrichtung 1 eine Einrichtung 9 zum Einlesen eines Endzeitpunkts 11 der Blutbehandlungssitzung auf. Dieses Einlesen kann mittels eines Daten-Netzwerkes und einem an anderer Stelle befindlichen Einlesegerätes erfolgen. Auch die Eingabe in das System mittels Tastatur oder Touchscreen kann erfindungsgemäß eingelesen werden.

Zum Einlesen der Behandlungsdauer 15 der Blutbehandlungssitzung in die Vorrichtung 1 weist diese weiterhin eine Einrichtung 13 auf. Das Einlesen kann mittels gleicher oder ähnlicher Prinzipien wie das Einlesen des Endzeitpunkts 11 erfolgen.

Die eingegebenen Daten werden in diesem Ausführungsbeispiel mittels eines Prozessors 17 verarbeitet. Das Ergebnis der Berechnung durch den Prozessors 17 ist ein positiver oder ein negativer Zeitpuffer. Dieser Zeitpuffer wird mittels einer Einrichtung 19 der Vorrichtung 1 ausgegeben oder dargestellt.

Alternativ (hier nicht dargestellt) kann die Einrichtung 19 außerhalb der Vorrichtung 1 angeordnet sein, oder das Ergebnis der Berechnung an eine außerhalb der Vorrichtung 1 angeordnete weitere Einrichtung wie beispielsweise einen PDA (personal digital assistant), einen Blackberry, einen mobilen, kabellosen Kommunikations- oder Organisationsassistenten und dergleichen übertragen werden.

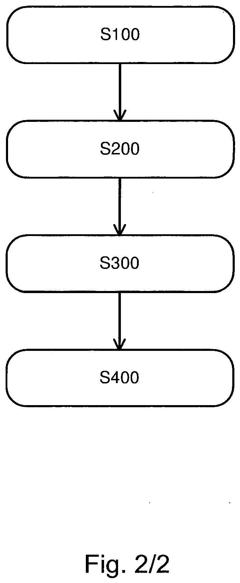

**Fig. 2** zeigt in einem Flussdiagramm exemplarisch die Schritte des offenbarten Verfahrens zum Quantifizieren und Darstellen eines Zeitpuffers in einer Ausführungsform.

In einem ersten Schritt S100 wird die Behandlungsdauer in die Vorrichtung 1 eingelesen, beispielsweise mittels der Einrichtung zum Einlesen der Behandlungsdauer 7, wie beispielsweise einem Kartenlesegerät, von der Patientenkarte 5.

In einem darauf folgenden zweiten Schritt S200 wird ein Endzeitpunkt 11 der Blutbehandlungssitzung durch die Vorrichtung 1, beispielsweise mittels der Einrichtung zum Einlesen des Endzeitpunkts 9, eingelesen. Ebenso kann der Endzeitpunkt mittels eines Daten-Netzwerkes von einem anderen Computer, einem Speicher usw. eingelesen werden.

Die Reihenfolge der Schritte S100 und S200 ist dabei beliebig.

In einem weiteren Schritt S300 wird ein positiver oder negativer Zeitpuffer durch die Vorrichtung 1 berechnet. Die Berechnung erfolgt beispielsweise mittels eines Prozessors 17 in der Vorrichtung 1. In die Berechnung gehen die aktuelle (Uhr-)Zeit, die im Schritt S100 eingelesene Behandlungsdauer und der im Schritt S200 eingelesene Endzeitpunkt der Blutbehandlungssitzung ein.

Wenn beispielsweise die Berechnung ergibt, dass mit der aktuellen Zeit als potentiellem Start der Blutbehandlungssitzung zusammen mit der eingelesenen Behandlungsdauer genau der eingelesene Endzeitpunkt erreicht wird, so ist der Zeitpuffer weder positiv noch negativ.

Gleichwohl kann mittels der Vorrichtung 1 angezeigt werden, dass Eile besteht, um mit der Behandlungssitzung des Patienten zu beginnen.

Ergibt jedoch die Berechnung, dass zwischen der aktuellen Zeit als potentiellem Startzeitpunkt der Blutbehandlungssitzung zusammen mit der eingelesenen Behandlungsdauer noch ein Zeitpuffer besteht, so ist der Zeitpuffer positiv. Ein positiver Zeitpuffer kann beispielsweise dazu genutzt werden, die Behandlungssitzung eines anderer Patienten, für welchen ein negativer Zeitpuffer errechnet wurde, einzuleiten. Alternativ kann auch die Behandlungsdauer kurzfristig um die Größe des positiven Zeitpuffers verlängert werden, und die Behandlungssitzung vorzeitig begonnen werden. Der Zeitpuffer des Patienten wird an die derart geänderte Behandlungsdauer angepasst.

Ein negativer Zeitpuffer wird ermittelt, wenn beispielsweise der eingelesene Endzeitpunkt überschritten wird, selbst wenn bei Einhalten der eingelesenen Behandlungsdauer die Blutbehandlungssitzung sofort begonnen wird. Ein negativer Zeitpuffer kann beispielsweise zu einer verkürzten Behandlungsdauer führen.

In einem weiteren Schritt S400 wird der Zeitpuffer angezeigt. In Fig. 1 wird der Zeitpuffer mittels einer Einrichtung 19 zum Ausgeben des Zeitpuffers angezeigt. Die Einrichtung 19 ist in diesem Ausführungsbeispiel als eine Art Ampel ausgeführt. Der obere Kreis kann ein rotes Signal anzeigen und damit einen negativen Zeitpuffer. Der untere Kreis kann ein grünes Signal anzeigen und damit einen positven Zeitpuffer. Der mittlere Kreis kann ein gelbes Signal anzeigen, der Zeitpuffer ist dann weder positiv noch negativ.

**Bezugszeichenliste**

| **Bezugszeichen** | **Beschreibung** |
|---|---|
| S100 | Schritt zum Einlesen der Behandlungsdauer |
| S200 | Schritt zum Einlesen des Endzeitpunkts |
| S300 | Berechnen des Zeitpuffers |
| S400 | Anzeige des Zeitpuffers |
| 1 | Vorrichtung |
| 2 | Blutbehandlungsvorrichtung |
| 3 | Patient |
| 5 | Patientenkarte |
| 7 | Einrichtung zum Einlesen der Behandlungsdauer |
| 9 | Einrichtung zum Einlesen des Endzeitpunkts |
| 11 | Endzeitpunkt der Blutbehandlungssitzung |
| 13 | Einrichtung zum Einlesen der Behandlungsdauer |
| 15 | Behandlungsdauer der Blutbehandlungssitzung |
| 17 | Einrichtung zum Berechnen des Zeitpuffers |
| 19 | Einrichtung zum Ausgeben des Zeitpuffers |

## Patentansprüche

1. Blutbehandlungsvorrichtung (2), welche wenigstens eine Vorrichtung (1), konfiguriert zum Quantifizieren und zum Darstellen eines Zeitpuffers oder einer Eilbedürftigkeit wenigstens eines Patienten (3) bei einer Blutbehandlungssitzung, aufweist, wobei die Vorrichtung (1) aufweist:
- eine Einrichtung (7) zum Einlesen einer Behandlungsdauer (15);
- eine Einrichtung (9) zum wenigstens einmaligen Einlesen eines angestrebten Endzeitpunkts (11) der Blutbehandlungssitzung;
- eine Einrichtung (17) zum wenigstens einmaligen Berechnen oder Ermitteln oder Klassifizieren des Zeitpuffers oder der Eilbedürftigkeit, basierend wenigstens auf der aktuellen Zeit, der Behandlungsdauer (15) und dem Endzeitpunkt (11); und
- eine Einrichtung (19) zum wenigstens einmaligen Ausgeben eines Signals, welches den Zeitpuffer oder die Eilbedürftigkeit angibt oder hierfür codiert.

2. Blutbehandlungsvorrichtung (2) nach Anspruch 1, wobei die Vorrichtung (1) aufweist:
- eine Einrichtung konfiguriert zum Bestimmen des Zeitpuffers oder des Beginns der Blutbehandlungssitzung unter Berücksichtigung der Gesamtdauer von weiteren, zur Durchführung der Blutbehandlung oder der Blutbehandlungssitzung erforderlichen Schritten, während welcher keine Behandlung des Bluts erfolgt, oder zum Korrigieren des Signals, welches den Zeitpuffers oder die Eilbedürftigkeit angibt oder hierfür codiert um die zur Durchführung der weiteren Schritte erforderliche Zeit.

3. Blutbehandlungsvorrichtung (2) nach einem der vorangegangenen Ansprüche, wobei die Vorrichtung (1) aufweist:
- eine Einrichtung konfiguriert zum Einlesen einer dem Patienten (3) verschriebenen Gesamtbehandlungsdauer, welche über eine vorbestimmte Anzahl aufeinander folgender Behandlungssitzungen verteilt wurde;
- eine Einrichtung konfiguriert zum Aufsummieren der Dauern (15), während welcher der Patient (3) bei vorangegangenen Behandlungssitzungen tatsächlich behandelt wurde; und
- eine Einrichtung zum Festlegen oder Abändern der eingelesenen Behandlungsdauer (15) der anstehenden Blutbehandlungssitzung unter Berücksichtigung der eingelesenen Gesamtbehandlungsdauer und der aufsummierten Dauern, während welcher der Patient (3) tatsächlich behandelt wurde.

4. Blutbehandlungsvorrichtung (2) nach einem der vorangegangenen Ansprüche, wobei die Vorrichtung (1) eine Einrichtung aufweist, welche konfiguriert ist zum Eingeben einer gegenüber der eingelesenen Behandlungsdauer (15) verkürzten oder verlängerten Behandlungsdauer, wobei die Vorrichtung (1) konfiguriert ist zum Berechnen oder Ermitteln oder Klassifizieren des Zeitpuffers oder der Eilbedürftigkeit, basierend wenigstens auf der aktuellen Zeit, der verkürzten oder verlängerten Behandlungsdauer (15) und dem Endzeitpunkt (11).

5. Blutbehandlungsvorrichtung (2) nach einem der vorangegangenen Ansprüche, wobei die Vorrichtung (1) aufweist:
- eine Einrichtung zum mehrfachen Abfragen oder Identifizieren eines Fortgangs der Behandlung des Patienten (3) mittels einer Abfrageeinrichtung während der Behandlungssitzung durch Auslesen oder Identifizieren von durch die Behandlungsvorrichtung (2) vorgenommenen oder initiierten Unterbrechungen der Behandlung des Patienten (3);
- eine Einrichtung zum Ermitteln der Gesamtzeitdauer der identifizierten Unterbrechungen der Behandlung mittels der Abfrageeinrichtung; und
- eine Einrichtung konfiguriert zum Berechnen oder Ermitteln oder Klassifizieren des Zeitpuffers oder der Eilbedürftigkeit, basierend wenigstens auf der aktuellen Zeit, der Behandlungsdauer (15), der Gesamtzeitdauer der identifizierten Unterbrechungen und des Endzeitpunkts (11).

6. Blutbehandlungsvorrichtung (2) nach einem der vorangegangenen Ansprüche, wobei die Vorrichtung (1) eine Einrichtung aufweist, konfiguriert zum Vergleichen von Zeitpuffern oder Eilbedürftigkeiten von mehreren Patienten miteinander und zum Festlegen einer Behandlungsreihenfolge zwischen diesen Patienten (3) aufgrund des Ergebnisses des Vergleichs.

## Claims

1. A blood treatment apparatus (2) comprising at least one apparatus (1) configured to quantify and to display a time buffer or an urgency need of at least one patient (3) at a blood treatment session, wherein the apparatus (1) comprises:
- a device (7) for reading in a treatment duration (15);
- a device (9) for, at least once, reading in a target end time point (11) of the blood treatment session;
- a device (17) for, at least once, calculating or determining or classifying the time buffer or the urgency need, on the basis of at least the current time, the treatment duration (15) and the end time point (11); and
- a device (19) for, at least once, outputting a signal indicating or encoding the time buffer or the urgency need to that end.

2. The blood treatment apparatus (2) according to claim 1,
wherein the apparatus (1) comprises:
- a device configured to specify the time buffer or the start of the blood treatment session taking into account the total duration of further steps required to perform the blood treatment or the blood treatment session during which no treatment of the blood takes place, or to correct the signal indicating the time buffer or the urgency need or encoded to that end, by the time required to perform the further steps.

3. The blood treatment apparatus (2) according to anyone of the preceding claims, wherein the apparatus (1) comprises:
- a device configured to read in a total treatment duration prescribed for the patient (3), which was distributed over a predetermined number of successive treatment sessions;
- a device configured to sum up the durations (15) during which the patient (3) was actually treated during previous treatment sessions; and
- a device for setting or changing the read-in treatment duration (15) of the upcoming blood treatment session, taking into account the total read-in treatment duration and the summed up durations during which the patient (3) was actually treated.

4. The blood treatment apparatus (2) according to anyone of the preceding claims, wherein the apparatus (1) comprises a device configured for input of a treatment duration which is shortened or extended relative to the read-in treatment duration (15), wherein the device (1) is configured to calculate or determine or classify the time buffer or the urgency need on the basis of at least the current time, the shortened or extended duration (15) and the end time point (11).

5. The blood treatment apparatus (2) according to anyone of the preceding claims, wherein the apparatus (1) comprises:
- a device for repeatedly querying or for identifying a progress of the treatment of the patient (3), during the treatment session by means of an interrogator, which reads or identifies interruptions of the treatment of the patient (3) performed or initiated by the treatment device (2);
- a device for determining the total duration of the interruptions of the treatment identified by the interrogator; and
- a device configured to calculate or determine or classify the time buffer or the urgency need on the basis of at least the current time, the treatment duration (15), the total time of the identified interruptions and the end time point (11).

6. The blood treatment apparatus (2) according anyone of the preceding claims, wherein the apparatus (1) comprises a device configured to compare time buffers or urgency needs of several patients with each other and to set a treatment order between said patients (3) on the basis of the result of the comparison.

## Revendications

1. Un appareil de traitement du sang (2), comprenant au moins un appareil (1), configuré de façon à quantifier et à représenter une durée-tampon ou une urgence d'au moins un patient (3) lors d'une séance de traitement du sang, où l'appareil (1) comprend :
- un dispositif (7) prévu pour l'entrée d'une durée de traitement (15);
- un dispositif (9) prévu pour l'entrée, au moins une fois, de l'heure de fin (11) souhaitée de la séance de traitement du sang;
- un dispositif (17) prévu pour calculer ou déterminer ou classifier au moins une fois la durée-tampon ou l'urgence en fonction au moins de l'heure actuelle, de la durée de traitement (15) ainsi que de l'heure de fin (11); et
- un dispositif (19) prévu pour émettre au moins une fois un signal indiquant ou encodant à cette fin la durée-tampon ou l'urgence.

2. L'appareil de traitement du sang (2) selon la première revendication, où l'appareil (1) comprend :
- un dispositif configuré de façon à déterminer la durée-tampon ou le début de la séance de traitement du sang, en tenant compte de la durée totale d'étapes supplémentaires nécessaires à la réalisation du traitement du sang ou de la séance de traitement du sang, étapes au cours desquelles un traitement du sang n'a pas lieu, ou configuré de façon à corriger le signal indiquant ou encodant à cette fin la durée-tampon ou l'urgence du temps nécessaire à la réalisation des étapes supplémentaires.

3. L'appareil de traitement du sang (2) selon l'une quelconque des revendications précédentes, où l'appareil (1) comprend :
- un dispositif configuré de façon à lire une durée totale de traitement prescrite au patient (3), laquelle a été répartie sur un nombre prédéterminé de séances de traitement successives;
- un dispositif configuré de façon à additionner les périodes (15) au cours desquelles le patient (3) a été effectivement traité lors des séances de traitement précédentes; et
- un dispositif configuré de façon à fixer ou à modifier la durée de traitement (15) entrée de la prochaine séance de traitement du sang, en tenant compte de la durée totale du traitement entrée et des périodes additionnées au cours desquelles le patient (3) a été effectivement traité.

4. L'appareil de traitement du sang (2) selon l'une quelconque des revendications précédentes, où l'appareil (1) comprend un dispositif configuré de façon à saisir une durée de traitement raccourcie ou prolongée par rapport à la durée de traitement (15) entrée, où l'appareil (1) est configuré de façon à calculer ou à déterminer ou à classifier la durée-tampon ou l'urgence en fonction au moins de l'heure actuelle, de la durée de traitement (15) raccourcie ou prolongée ainsi que de l'heure de fin (11).

5. L'appareil de traitement du sang (2) selon l'une quelconque des revendications précédentes, où l'appareil (1) comprend :
- un dispositif prévu pour, à plusieurs reprises, interroger ou identifier une évolution du traitement du patient (3) au moyen d'un dispositif d'interrogation, qui, lors de la séance de traitement, extrait ou identifie les interruptions du traitement du patient (3) effectuées ou initiées par l'appareil de traitement (2);
- un dispositif prévu pour déterminer la durée totale des interruptions du traitement identifiées au moyen du dispositif d'interrogation; et
- un dispositif configuré de façon à calculer ou à déterminer ou à classifier la durée-tampon ou l'urgence en fonction au moins de l'heure actuelle, de la durée du traitement (15), de la durée totale des interruptions identifiées et de l'heure de fin (11).

6. L'appareil de traitement du sang (2) selon l'une quelconque des revendications précédentes, où l'appareil (1) comprend un dispositif configuré de façon à comparer les durées-tampon ou les urgences de plusieurs patients (3) les uns avec les autres et à fixer un ordre de traitement entre ces patients (3) sur la base du résultat de la comparaison.
